# EUROPEAN PATENT APPLICATION

(11) **EP 4 703 330 A1**
(43) Date of publication of application: **04.03.2026**
(21) Application number: 24797002.3
(22) Date of filing: 23.04.2024
(51) Int. Cl.: C03C 3/089, A61K 6/15, A61K 6/836, C03C 3/062, C03C 3/076

(54) **GLASS COMPOSITION AND X-RAY RADIOPAQUE COMPOSITION**

(30) Priority: 28.04.2023 JP 2023074448
(71) Applicant: GC R&D Corporation, Tokyo 174-8585 (JP)
(72) Inventor: FUNAYAMA, Naoya, Tokyo 174-8585 (JP); MIYAKE, Takahiro, Tokyo 174-8585 (JP)
(74) Representative: Müller-Boré & Partner Patentanwälte PartG mbB
(86) International application number: PCT/JP2024/015852
(87) International publication number: WO 2024/225256

(57) **Abstract**

A glass composition contains rubidium (Rb).

## Description

### TECHNICAL FIELD

The present invention relates to glass composition and an X-ray contrast composition.

### BACKGROUND ART

X-ray-opaque glass containing Cs₂O, BaO, and/or SnO₂, and any desirable type of fluorine is known in the field of dentistry (for example, PTL 1).

### CITATION LIST

### PATENT LITERATURE

PTL 1: Japanese Patent Application Laid-Open Publication No. 2019-131457

### SUMMARY OF THE INVENTION

### TECHNICAL PROBLEM

Conventionally, glass having a higher X-ray contrast property tends to have a higher refractive index. Therefore, it is difficult to achieve both of a high X-ray contrast and a low refractive index.

An object of the present invention is to provide a glass composition from which glass achieving both a high X-ray contrast property and a low refractive index can be obtained.

### SOLUTION TO THE PROBLEM

The glass composition according to an embodiment of the present invention contains rubidium (Rb).

### ADVANTAGEOUS EFFECTS OF THE INVENTION

According to one embodiment of the present invention, it is possible to provide a glass composition from which glass having both a high X-ray contrast property and a low refractive index can be obtained.

### DETAILED DESCRIPTION OF THE INVENTION

Embodiments of the present invention will be described in detail below.

A glass composition according to the present embodiment contains rubidium (Rb).

Rubidium (Rb) has the atomic number (number of electrons) of 37, and has approximately the same atomic number (number of electrons) as that of strontium (Sr), which has the atomic number (number of electrons) of 38 and exhibits a high X-ray contrast property in glass. Therefore, rubidium (Rb) exhibits as high an X-ray contrast property as that of strontium (Sr) in glass.

Here, the X-ray contrast property is a property that changes the density of an X-ray image of an object, such as a biological tissue, a material, and the like, depending on the amount of X-rays absorbed by the object, to enable the object to be visible or distinctive. In particular, in the dental treatment, the area treated with dental materials, such as dental fillers, needs to be clarified after the treatment, and the dental materials need therefore to have an X-ray contrast property. Resin matrices of dental materials are organic substances and do not hence have an X-ray contrast property. Therefore, fillers made of inorganic substances, such as glass and the like, are responsible for the X-ray contrast property. Therefore, the X-ray contrast property is an important property in the dental glass.

Rubidium (Rb) has an effective ionic radius of 152 pm, which is greater than that of strontium (Sr), which has an effective ionic radius of 118 pm. The greater the effective ionic radius, the lower the electron density becomes or the sparser electrons become in the atom relatively. Therefore, rubidium (Rb) can inhibit an increase in the refractive index of glass better than strontium (Sr).

Furthermore, rubidium (Rb) having an effective ionic radius of 152 pm has an effective ionic radius smaller than that of cesium (Cs), which is an alkali metal as is rubidium (Rb) and has an effective ionic radius of 167 pm. Among homologous alkali metals, there is a tendency that those that have a smaller effective ionic radius have a greater single-bonding strength with oxygen, and have a higher chemical stability. Therefore, glass with rubidium (Rb) tends to experience less chemical durability deterioration than glass with cesium (Cs) does. Examples of the chemical durability include water resistance.

Rubidium (Rb) can exist as an oxide of rubidium (Rb) (rubidium oxide (Rb₂O)) in a glass composition.

The content of rubidium (Rb) in terms of an oxide is not particularly limited, and is, for example, 3% by mass or greater, preferably 5% by mass or greater, and more preferably 7% by mass or greater. The content of rubidium (Rb) in terms of an oxide indicates the mass content ratio of rubidium oxide (Rb₂O), which is an oxide of rubidium (Rb), in a glass composition.

The upper limit of the content of rubidium (Rb) in terms of an oxide is not particularly limited. Yet, the content of rubidium (Rb) is preferably 60% by mass or less, more preferably 50% by mass or less, and more preferably 40% by mass or less from the viewpoint of inhibiting deterioration of the chemical durability of the glass composition.

In the present embodiment, the glass composition containing rubidium (Rb) can enhance the X-ray contrast property of the glass to be obtained. Moreover, it is easy to control the refractive index of the glass to be obtained, because the glass would undergo little refractive index increase while maintaining a high X-ray contrast property. Furthermore, the glass composition containing rubidium (Rb) makes the chemical durability of the glass to be obtained less likely to deteriorate.

As long as the glass composition of the present embodiment contains rubidium (Rb), the type of the glass composition is not particularly limited. Examples of the type of the glass composition include silicate glass, borate glass, phosphate glass, borosilicate glass, silicate-phosphate glass, fluorophosphate glass, sulfate-phosphate glass, soda lime glass, aluminosilicate glass, aluminoborosilicate glass, and the like.

Among these, silicate glass, borate glass, and borosilicate glass are preferable as the type of glass composition. The main component of silicate glass is silicon (Si).

That is, it is preferable that the glass composition of the present embodiment contains silicon (Si). Silicon (Si) may exist as an oxide of silicon (Si) (silicon oxide (SiO₂)) in the glass composition.

The content of silicon (Si) is not particularly limited, yet is preferably 10% by mass or greater, more preferably 15% by mass or greater, and yet more preferably 20% by mass or greater in terms of an oxide. The content of silicon (Si) in terms of oxide indicates the mass content ratio of silicon oxide (SiO₂), which is an oxide of silicon (Si), in the glass composition.

When the content of silicon (Si) in terms of an oxide is 10% by mass or greater, it is possible to adjust the viscosity of the glass composition, and to enhance the mechanical strength of the glass composition.

The upper limit of the content of silicon (Si) in terms of an oxide is not particularly limited. However, because the glass melt becomes hard when silicon (Si) in the glass composition increases, the content is preferably 90% by mass or less, more preferably 85% by mass or less, and yet more preferably 80% by mass or less from the viewpoint of reducing the production cost.

The glass composition of the present embodiment may further contain cesium (Cs).

Since cesium (Cs) has the atomic number (number of electrons) of 55 and has approximately the same atomic number (number of electrons) as that of barium (Ba), which has the atomic number (number of electrons) of 56, cesium exhibits as high an X-ray contrast property as that of barium (Ba). Therefore, mixing cesium (Cs) with rubidium (Rb) can also enhance the X-ray contrast property of the glass to be obtained.

Moreover, since cesium (Cs) has an effective ionic radius of 167 pm and is larger than barium (Ba), which has an effective ionic radius of 135 pm (i.e., cesium (Cs) has a lower electron density or has electrons more sparsely in the atom), cesium can inhibit an increase in the refractive index better than barium (Ba). Therefore, mixing cesium (Cs) with rubidium (Rb) can also inhibit an increase in the refractive index of the glass to be obtained, while maintaining a high X-ray contrast property.

Furthermore, although cesium (Cs) is inferior to rubidium (Rb) in chemical durability, mixing cesium (Cs) with rubidium (Rb) does not cause a decrease in the chemical durability due to the mixing of rubidium (Rb). Therefore, by replacing a part of rubidium (Rb), which has a relatively high raw material cost, with cesium (Cs), which has a relatively low raw material cost, it is possible to avoid an increase in the manufacturing cost of the glass composition.

Cesium (Cs) can exist as an oxide of cesium (Cs) (cesium oxide (Cs₂O)) in the glass composition.

When cesium (Cs) is contained, the content of cesium (Cs) is not particularly limited, and is, for example, 3% by mass or greater, preferably 5% by mass or greater, and more preferably 10% by mass or greater in terms of an oxide. The content of cesium (Cs) in terms of an oxide indicates the mass content ratio of cesium (Cs) oxide (Cs₂O), which is an oxide of cesium (Cs), in the glass composition.

When the content of cesium (Cs) in terms of an oxide is 3% by mass or greater, it is possible to enhance the X-ray contrast property of the glass to be obtained, and to inhibit an increase in the refractive index, synergistically with rubidium (Rb). The upper limit of the content of cesium (Cs) in terms of an oxide is not particularly limited, yet is preferably 40% by mass or less, more preferably 30% by mass or less, and more preferably 20% by mass or less from the viewpoint of not reducing the rubidium (Rb) content.

The glass composition of the present embodiment may further contain potassium (K). Potassium (K) may exist as an oxide of potassium (K) (potassium oxide (K₂O)) in the glass composition.

When potassium (K) is contained, the content of potassium (K) is 1 % by mass or greater and 15% by mass or less, preferably 3% by mass or greater and 12% by mass or less, and yet more preferably 5% by mass or greater and 10% by mass or less in terms of an oxide. The content of potassium (K) in terms of an oxide indicates the mass content ratio of potassium oxide (K₂O), which is an oxide of potassium (K), in the glass composition.

When potassium (K) is contained and the content of potassium (K) in terms of an oxide is 1% by mass, it is possible to lower the melting temperature of the glass composition, and to therefore facilitate manufacture or processing of the glass composition, which can hence be applied as materials in various fields. In addition, when the content of potassium (K) in terms of an oxide is 15 mass% or less, it is possible to inhibit deterioration of the chemical durability of the glass composition.

The glass composition of the present embodiment may further contain sodium (Na). Sodium (Na) may exist in the glass composition as an oxide of sodium (Na) (sodium oxide (Na₂O)).

When sodium (Na) is contained, the content of sodium (Na) is 1% by mass or greater and 15% by mass or less, preferably 1.5% by mass or greater and 12% by mass or less, and more preferably 2% by mass or greater and 10% by mass or less in terms of an oxide. The content of sodium (Na) in terms of an oxide indicates the mass content ratio of sodium oxide (Na₂O), which is an oxide of sodium (Na), in the glass composition.

When sodium (Na) is contained and the content of sodium (Na) in terms of an oxide is 1% by mass, it is possible to lower the melting temperature of the glass composition, and to facilitate manufacture or processing of the glass composition, which can hence be applied as materials in various fields. In addition, when the content of sodium (Na) in terms of an oxide is 15% by mass or less, it is possible to inhibit deterioration of the chemical durability of the glass composition.

It is preferable that the glass composition of the present embodiment is free of at least one selected from the group consisting of potassium (K) and sodium (Na) from the viewpoint of reliably inhibiting deterioration of the chemical durability of the glass composition. In this specification, the phrase "free of at least one selected from the group consisting of potassium (K) and sodium (Na)" means that the glass composition does not substantially contain at least one selected from the group consisting of potassium (K) and sodium (Na) except for unavoidable impurities.

When potassium (K) is contained in the glass composition as an unavoidable impurity, the content of potassium (K) is 0.5% by mass or less, preferably 0.3% by mass or less, and more preferably 0.1% by mass or less in terms of an oxide.

When sodium (Na) is contained in the glass composition as an unavoidable impurity, the content of sodium (Na) is 0.5% by or less, preferably 0.3 mass% or less, and more preferably 0.1% by mass or less in terms of an oxide.

The glass composition of the present embodiment may contain other elements as long as the purpose of the present invention is not compromised. Examples of the other elements include boron (B), fluorine (F), aluminum (Al), zinc (Zn), strontium (Sr), zirconium (Zr), tin (Sn), barium (Ba), lanthanum (La), gadolinium (Gd), and the like from the viewpoint of improving the X-ray contrast property or the chemical durability. The other elements, except fluorine (F), may exist as oxides in the glass composition. The content of the other elements is not particularly limited and they can be contained in the glass composition in any desirable masses.

Boron (B) can exist as an oxide of boron (B) (boron oxide (B₂O₃)) in the glass composition.

When boron (B) is contained, the content of boron (B) is not particularly limited, yet is preferably 40% by mass or less, more preferably 35% by mass or less, and yet more preferably 30% by mass or less in terms of an oxide. The content of boron (B) in terms of an oxide indicates the mass content ratio of boron oxide (B₂O₃), which is an oxide of boron (B), in the glass composition.

When the content of boron (B) in terms of an oxide is 40% by mass or less, it is possible to lower the melting point of the glass of the glass composition, and to facilitate manufacture. When boron (B) is contained, the lower limit of the content of boron (B) in terms of an oxide is not particularly limited, yet is preferably 1% by mass or greater, more preferably 2% by mass or greater, and yet more preferably 3% by mass or greater from the viewpoint of the chemical durability and ease of manufacturing of the glass composition.

Fluorine (F) may exist as a fluoride or a fluoride salt in the glass composition.

When fluorine (F) is contained, the content of fluorine (F) is not particularly limited, yet is preferably 6% by mass or less, more preferably 5.5% by mass or less, and yet more preferably 5% by mass or less. The content of fluorine (F) indicates the mass content ratio of fluorine (F) as a simple substance in the glass composition.

When the content of fluorine (F) is 6% by mass or less, it is possible to adjust the refractive index of the glass to be obtained. When fluorine (F) is contained, the lower limit of the content of fluorine (F) is not particularly limited, yet is preferably 0.1% by mass or greater, more preferably 1% by mass or greater, and yet more preferably 2% by mass or greater from the viewpoint of reducing the viscosity of the glass composition and improving the handleability of the glass composition.

Aluminum (Al) may exist as an oxide of aluminum (Al) (aluminum oxide (Al₂O₃)) in the glass composition.

When aluminum (Al) is contained, the content of aluminum (Al) is not particularly limited, yet is preferably 35% by mass or less, more preferably 30% by mass or less, and yet more preferably 25% by mass or less in terms of an oxide. The content of aluminum (Al) in terms of an oxide indicates the mass content ratio of aluminum oxide (Al₂O₃), which is an oxide of aluminum (Al), in the glass composition.

When the content of aluminum (Al) in terms of an oxide is 35% by mass or less, it is possible to improve the chemical durability of the glass composition and to inhibit devitrification of the glass. When aluminum (Al) is contained, the lower limit of the content of aluminum (Al) in terms of an oxide is not particularly limited, yet is preferably 0.1% by mass or greater, more preferably 0.5% by mass or greater, and yet more preferably 1% by mass or greater from the viewpoint of the chemical durability of the glass composition and inhibiting devitrification of the glass.

Zinc (Zn) may exist in the glass composition as an oxide of zinc (Zn) (zinc oxide (ZnO)).

When zinc (Zn) is contained, the content of zinc (Zn) is not particularly limited, yet is preferably 20% by mass or less, more preferably 15% by mass or less, and yet more preferably 10% by mass or less in terms of an oxide. The content of zinc (Zn) in terms of an oxide indicates the mass content ratio of zinc oxide (ZnO), which is an oxide of zinc (Zn), in the glass composition.

When the content of zinc (Zn) in terms of an oxide is 20% by mass or less, it is possible to inhibit devitrification of the glass to be obtained without deteriorating the chemical durability thereof, and to facilitate manufacture. The lower limit of the content of zinc (Zn) in terms of an oxide when zinc (Zn) is contained is not particularly limited, yet preferably 0.1% by mass or greater, more preferably 0.5% by mass or greater, and yet more preferably 1% by mass or greater from the viewpoint of the chemical durability of the glass composition and inhibiting devitrification of the glass.

Strontium (Sr) can exist as an oxide of strontium (Sr) (strontium oxide (SrO)) in the glass composition.

When strontium (Sr) is contained, the content of strontium (Sr) is not particularly limited, yet is preferably 20% by mass or less, more preferably 15% by mass or less, and yet more preferably 10% by mass or less in terms of an oxide. The content of strontium (Sr) in terms of an oxide indicates the mass content ratio of strontium oxide (SrO), which is an oxide of strontium (Sr), in the glass composition.

When the content of strontium (Sr) in terms of an oxide is 20% by mass or less, it is possible to enhance the X-ray contrast property of the glass to be obtained while maintaining a low refractive index thereof. The lower limit of the content of strontium (Sr) in terms of an oxide when strontium (Sr) is contained is not particularly limited, yet is preferably 0.1% by mass or greater, more preferably 0.5% by mass or greater, and yet more preferably 1% by mass or greater from the viewpoint of enhancing the X-ray contrast property while maintaining a low refractive index.

Zirconium (Zr) may exist as an oxide of zirconium (Zr) (zirconium oxide (ZrO₂)) in the glass composition.

When zirconium (Zr) is contained, the content of zirconium (Zr) is not particularly limited, yet is preferably 20% by mass or less, more preferably 15% by mass or less, and yet more preferably 10% by mass or less in terms of an oxide. The content of zirconium (Zr) in terms of an oxide indicates the mass content ratio of zirconium oxide (ZrO₂), which is an oxide of zirconium (Zr), in the glass composition.

When the content of zirconium (Zr) in terms of an oxide is 20% by mass or less, it is possible to improve the X-ray contrast property of the glass to be obtained and enhance the chemical durability thereof while maintaining the glass melting property. The lower limit of the content of zirconium (Zr) in terms of an oxide when zirconium (Zr) is contained is not particularly limited, yet is preferably 0.5% by mass or greater, more preferably 1% by mass or greater, and yet more preferably 3% by mass or greater from the viewpoint of enhancing the chemical durability while maintaining the X-ray contrast property.

Tin (Sn) can exist as an oxide of tin (Sn) (tin dioxide (SnO₂)) in the glass composition.

When tin (Sn) is contained, the content of tin (Sn) is not particularly limited, yet is preferably 25% by mass or less, more preferably 20 mass% or less, and yet more preferably 15% by mass or less in terms of an oxide. The content of tin (Sn) in terms of an oxide indicates the mass content ratio of tin oxide (SnO₂), which is an oxide of tin (Sn), in the glass composition.

When the content of tin (Sn) in terms of an oxide is 25% by mass or less, it is possible to improve the X-ray contrast property of the glass to be obtained and enhance the chemical durability thereof while maintaining the melting property of the glass. The lower limit of the content of tin (Sn) in terms of an oxide when tin (Sn) is contained is not particularly limited, yet is preferably 1% by mass or greater, more preferably 3% by mass or greater, and yet more preferably 5% by mass or greater from the viewpoint of enhancing the chemical durability while maintaining the X-ray contrast property.

Barium (Ba) may exist as an oxide of barium (Ba) (barium oxide (BaO)) in the glass composition.

When barium (Ba) is contained, the content of barium (Ba) is not particularly limited, yet is preferably 30% by mass or less, more preferably 25% by mass or less, and yet more preferably 20% by mass or less in terms of an oxide. The content of barium (Ba) in terms of an oxide indicates the mass content ratio of barium oxide (BaO), which is an oxide of barium (Ba), in the glass composition.

When the content of barium (Ba) in terms of an oxide is 30% by mass or less, it is possible to improve the X-ray contrast property of the glass to be obtained while inhibiting deterioration of the chemical durability. The lower limit of the content of barium (Ba) in terms of an oxide when barium (Ba) is contained is not particularly limited, yet is preferably 1% by mass or greater, more preferably 5% by mass or greater, and yet more preferably 10% by mass or greater from the viewpoint of imparting a high X-ray contrast property.

Lanthanum (La) can exist as an oxide of lanthanum (La) (lanthanum oxide (La₂O₃)) in the glass composition.

When lanthanum (La) is contained, the content of lanthanum (La) is not particularly limited, yet is preferably 40% by mass or less, more preferably 35% by mass or less, and yet more preferably 30% by mass or less in terms of an oxide. The content of lanthanum (La) in terms of an oxide indicates the mass content ratio of lanthanum oxide (La₂O₃), which is an oxide of lanthanum (La), in the glass composition.

When the content of lanthanum (La) in terms of an oxide is 40% by mass or less, it is possible to improve the X-ray contrast property of the glass to be obtained and enhance the chemical durability thereof while maintaining the melting property of the glass. The lower limit of the content of lanthanum (La) in terms of an oxide when lanthanum (La) is contained is not particularly limited, yet is preferably 1% by mass or greater, more preferably 5% by mass or greater, and yet more preferably 10% by mass or greater from the viewpoint of enhancing the chemical durability while maintaining the X-ray contrast property.

Gadolinium (Gd) can exist as an oxide of gadolinium (Gd) (gadolinium oxide (Gd₂O₃)).

When gadolinium (Gd) is contained, the content of gadolinium (Gd) is not particularly limited, yet is preferably 30% by mass or less, more preferably 25 % by mass or less, and yet more preferably 20% by mass or less in terms of an oxide. The content of gadolinium (Gd) in terms of an oxide indicates the mass content ratio of gadolinium oxide (Gd₂O₃), which is an oxide of gadolinium (Gd), in the glass composition.

When the content of gadolinium (Gd) in terms of an oxide is 30% by mass or less, it is possible to improve the X-ray contrast property of the glass to be obtained while maintaining the melting property of the glass. The lower limit of the content of gadolinium (Gd) in terms of an oxide when gadolinium (Gd) is contained is not particularly limited, yet is preferably 1% by mass or greater, more preferably 3% by mass or greater, and yet more preferably 5% by mass or greater from the viewpoint of imparting a high X-ray contrast property.

The glass composition of the present embodiment may contain other unavoidable impurities. Examples of the unavoidable impurities include nickel (Ni) and the like.

The form of the glass composition is not particularly limited, and may be, for example, powdery (or particulate), plate-like, columnar, string-like, or fibrous. When the form of the glass composition is powdery, it is easy to combine or mix the components of the glass composition. When the form of the glass composition is plate-like or columnar, the glass composition can be used as an optical element. Examples of optical elements include optical lenses, prisms, optical filters, and the like.

The particle size of the powdery glass composition is not particularly limited, yet the median diameter thereof is preferably 0.01 µm or greater and 20 µm or less, more preferably 0.02 µm or greater and 10 µm or less, and yet more preferably 0.1 µm or greater and 1 µm or less. Here, the particle size means the average particle size defined by the median diameter.

When the particle size of the glass composition is 0.01 µm or greater, the handleability of the glass composition when using the glass composition as a powder is improved. When the particle size of the glass composition is 20 µm or less, the mechanical strength of a dental composition obtained when the glass composition is applied to a dental material is improved.

The glass composition of the present embodiment can make the refractive index (nd) of glass containing the glass composition equal to or less than 1.564. In this specification, the refractive index (nd) means the refractive index of the D-line (light having a wavelength of 589 nm).

When the refractive index (nd) of the glass composition of the present embodiment is 1.564 or less, the refractive index difference from a dental resin when the glass composition is used as a dental composition is small, and the dental composition to be obtained has a high transparency. The lower limit of the refractive index (nd) is not particularly limited, yet a practical range thereof is preferably 1.40 or greater, more preferably 1.45 or greater, and yet more preferably 1.47 or greater.

The glass composition of the present embodiment can provide glass containing the glass composition with an X-ray contrast property of 200% or greater as a ratio with respect to aluminum. In this specification, the X-ray contrast property indicates the X-ray contrast property measured by an X-ray contrast property test method for dental materials according to ISO 4049:2019.

In the present embodiment, because the X-ray contrast property of glass containing the glass composition can be 200% or greater as ratio with respect to aluminum, it is possible to provide a glass composition exhibiting a good X-ray contrast property. The upper limit of the X-ray contrast property is not particularly limited, yet a practicable range thereof is preferably 1,000% or less, more preferably 900% or less, and yet more preferably 800% or less as a ratio with respect to aluminum.

The applications of the glass composition of the present embodiment are not particularly limited. Considering that the glass composition exhibits a high X-ray contrast property, the glass composition can be used as an X-ray contrast composition containing the glass composition.

With its high X-ray contrast property taken advantage of, the glass composition of the present embodiment can be used as a dental material or a dental composition. Examples of such a dental material or dental composition include a dental composite resin, a dental cement, a dental adhesive, a dental temporary sealing material, a dental primer, a dental coating material, a dental hard resin, a resin material for dental cutting processing, a dental temporary restorative material, a dental filling material, a dentifrice, and the like.

Since a dental composition of the present embodiment can impart both a high X-ray contrast property and a high transparency to glass containing itself, it is possible to obtain a dental composition that achieves both a good X-ray contrast property and excellent aesthetic property. In addition, since the dental composition of the present embodiment exhibits a good X-ray contrast property, it is possible to clarify a repaired region in a diagnosis or an examination based on an X-ray image after a treatment.

In addition, since the glass composition contained in the dental composition of the present embodiment is adjusted to a sufficiently suitable refractive index range, it can be suitably used for dental materials required to have a small refractive index from dental resins and have an aesthetic property (for example, a dental composite resin, a dental coating material, a dental hard resin, a resin material for dental cutting processing, and the like).

Furthermore, since the glass composition contained in the dental composition of the present embodiment has a good chemical durability, it can be suitably used for all types of dental materials that are applied in the oral cavity.

The glass composition of the present embodiment can also be applied to fields other than dental materials. Examples of such applications include optical elements, such as optical lenses, prisms, and optical filters, and the like, ultraviolet and infrared ray cutting glass, and radiation shielding glass, and the like.

### Examples

The present invention will further be described below based on Examples. In the following description, numerical values without units are on a mass-basis (% by mass) unless otherwise particularly specified.

### <Preparation of Glass>

Glass of each of Examples 1-1 to 1-3, Examples 2-1 to 2-4, Examples 3-1 to 3-2, and Examples 4-1 to 4-6, Comparative Examples 1-1 to 1-3, and Comparative Example 3-1 was prepared based on the compositions shown in Tables 1 to 4. A raw material batch obtained by mixing various raw materials was added into a platinum crucible, kept in an electric furnace preheated to 1,400°C to 1,500°C for 30 minutes to 60 minutes, and poured out into a carbon mold, to obtain a bulk body. Alternatively, after melting in a similar manner, a resulting glass melt was dropped into a water tank, to obtain water-granulated glass. The glass was pulverized in an alumina magnet mortar, to obtain glass powder having a median diameter (average particle diameter) of 20 µm to 300 µm.

The refractive index of the obtained glass powder (glass) was measured. The obtained glass bulk body was subjected to a thermal treatment in which it was subjected to temperature raising from room temperature up to 600°C at a temperature raising rate of 20°C/min in an electric furnace, kept at 600°C for 30 minutes, and then cooled slowly to room temperature. After the thermal treatment, the glass bulk body was sliced, to obtain a sliced piece.

### <Refractive Index>

A liquid having a specific refractive index and the prepared glass powder were mixed at a mass ratio of 1:1 and packed into a transparent tube. Next, the transparent tube was exposed to a white LED, to visually observe the color of light transmitted through the transparent tube. The refractive index of a liquid that caused green light to be transmitted through the transparent tube was adopted as the refractive index of the glass powder. When the liquid had a refractive index lower than that of the glass powder, light to be transmitted through the transparent tube would be blue. When the liquid had a refractive index higher than that of the glass powder, light to be transmitted through the transparent tube would be red or yellow. The refractive index of the glass was evaluated as being good when the refractive index was 1.564 or less, and evaluated as being bad when the refractive index was greater than 1.564. The results of the refractive index of the glass are shown in Tables 1 to 4.

### <X-Ray Contrast Property>

The X-ray contrast property (% vs. Al) of the obtained glass sliced piece was measured. The X-ray contrast property was measured by the X-ray contrast test property test method for dental materials according to ISO 4049:2019. Specifically, the optical density or gray value of a test piece (a plate-like glass composition) having a thickness Ts (unit: mm) was measured. Next, the thickness Ta (unit: mm) of an aluminum plate exhibiting the same optical density or gray value as that of the test piece having the thickness Ts was examined. Then, the ratio of Ta to Ts (unit: % vs. Al) was adopted as the value representing X-ray contrast property. The X-ray contrast property was evaluated as being good when the ratio with respect to aluminum was 200% or greater, and was evaluated as being bad when the ratio with respect to aluminum was less than 200%. The results of the refractive index of the glass are shown in Tables 1 to 4.

### <Water Resistance>

The glass powder (having a median diameter of 20 µm to 300 µm) was weighed out by 2.0 g. The glass powder was added into a Duran glass bottle (obtained from SCHOTT GmbH, "Duran" is a registered trademark) and shaken with addition of 50 mL of distilled water. The resulting product was put in a thermostatic bath preheated to 98°C while being kept in the glass bottle, and allowed to stand still for 1 hour. After being removed from the thermostatic bath, the resulting immersing liquid was allowed to cool to room temperature, and the electrical conductivity thereof was measured with an electrical conductivity meter (obtained from Horiba, Ltd.). In the water resistance test, the amount of ions eluted from the glass powder into the immersing liquid was measured and evaluated. The smaller the amount of eluted ions, that is, the smaller the value (unit: µS) evaluating the water resistance, the better water resistance the glass composition was evaluated to have. Specifically, the water resistance was evaluated as being good when it was 800 µS or less, and evaluated as being bad when it was greater than 800 µS. The results of the water resistance are shown in Tables 1, 3, and 4.

**[Table 1]**

| [mass%] | Ex. 1-1 | Ex. 1-2 | Ex. 1-3 | Comp. Ex. 1-1 | Comp. Ex. 1-2 | Comp. Ex. 1-3 |
|---|---|---|---|---|---|---|
| SiO₂ | 41.93 | 46.07 | 23.76 | 53.12 | 34.90 | 26.39 |
| B₂O₃ | 16.76 | 17.80 | 19.68 | 21.22 | 13.94 | 30.58 |
| Al₂O₃ | 9.82 | 13.04 | 23.05 | 12.43 | 8.17 | 6.40 |
| Na₂O | 0 | 3.96 | 7.01 | 13.23 | 0 | 7.78 |
| K₂O | 0 | 0 | 0 | 0 | 0 | 0 |
| Rb₂O | 31.49 | 19.13 | 10.57 | 0 | 0 | 0 |
| Cs₂O | 0 | 0 | 15.93 | 0 | 0 | 0 |
| BaO | 0 | 0 | 0 | 0 | 42.99 | 28.85 |
| Total | 100 | 100 | 100 | 100 | 100 | 100 |
| Refractive index | 1.493 | 1.485 | 1.503 | 1.493 | 1.565 | 1.555 |
| X-ray contrast property [% vs. Al] | 453 | 269 | 535 | 60 | 1,032 | 620 |
| Water resistance [µS] | 305 | 68 | 301 | 109 | 40 | 831 |

**[Table 2]**

| [mass%] | Ex. 2-1 | Ex. 2-2 | Ex. 2-3 | Ex. 2-4 |
|---|---|---|---|---|
| SiO₂ | 77.84 | 63.08 | 53.85 | 47.31 |
| B₂O₃ | 0 | 9.14 | 7.80 | 6.85 |
| Na₂O | 10.04 | 8.14 | 6.95 | 6.10 |
| Rb₂O | 12.12 | 19.64 | 31.40 | 39.74 |
| Cs₂O | 0 | 0 | 0 | 0 |
| Total | 100 | 100 | 100 | 100 |
| Refractive index | 1.485 | 1.505 | 1.508 | 1.517 |
| X-ray contrast property [% vs. Al] | 200 | 311 | 440 | 582 |

**[Table 3]**

| [mass%] | Ex. 3-1 | Ex. 3-2 | Comp. Ex. 3-1 |
|---|---|---|---|
| SiO₂ | 37.60 | 36.49 | 26.39 |
| B₂O₃ | 26.14 | 21.14 | 30.58 |
| Al₂O₃ | 6.38 | 12.39 | 6.40 |
| Na₂O | 0 | 0 | 7.78 |
| K₂O | 0 | 0 | 0 |
| Rb₂O | 23.40 | 11.35 | 0 |
| SrO | 6.48 | 0 | 0 |
| BaO | 0 | 18.63 | 28.85 |
| Total | 100 | 100 | 100 |
| Refractive index | 1.500 | 1.525 | 1.555 |
| X-ray contrast property [% vs. Al] | 409 | 677 | 620 |
| Water resistance [µS] | 643 | 252 | 831 |

**[Table 4]**

| [mass%] | Ex. 4-1 | Ex. 4-2 | Ex. 4-3 | Ex. 4-4 | Ex. 4-5 | Ex. 4-6 |
|---|---|---|---|---|---|---|
| SiO₂ | 30.57 | 54.60 | 41.28 | 30.77 | 36.07 | 40.28 |
| B₂O3 | 22.14 | 12.65 | 13.54 | 14.26 | 18.39 | 16.97 |
| Al₂O₃ | 19.46 | 5.70 | 10.58 | 5.22 | 3.67 | 14.91 |
| Na₂O | 2.37 | 8.67 | 9.64 | 0 | 7.44 | 2.27 |
| K₂O | 8.39 | 0 | 0 | 9.65 | 5.66 | 0 |
| Rb₂O | 11.89 | 7.84 | 16.97 | 13.40 | 15.71 | 22.79 |
| F | 0 | 0 | 0 | 0 | 0 | 2.78 |
| ZnO | 5.18 | 0 | 0 | 0 | 0 | 0 |
| SnO₂ | 0 | 10.54 | 0 | 0 | 0 | 0 |
| ZrO₂ | 0 | 0 | 7.99 | 0 | 0 | 0 |
| La₂O₃ | 0 | 0 | 0 | 26.70 | 0 | 0 |
| Gd₂O₃ | 0 | 0 | 0 | 0 | 13.06 | 0 |
| Total | 100 | 100 | 100 | 100 | 100 | 100 |
| Refractive index | 1.498 | 1.513 | 1.525 | 1.563 | 1.531 | 1.475 |
| X-ray contrast property [% vs. Al] | 237 | 321 | 372 | 852 | 486 | 323 |
| Water resistance [µS] | 344 | 53 | 63 | 94 | 546 | 342 |

According to Tables 1 to 4, the glass obtained from the glass compositions of Examples 1-1 to 1-3, Examples 3-1 to 3-2, and Examples 4-1 to 4-6 containing rubidium (Rb) was good in the refractive index, the X-ray contrast property, and the water resistance.

On the other hand, the glass obtained from the glass compositions of Comparative Examples 1-1 to 1-3 and Comparative Example 3-1 free of rubidium (Rb) was bad in at least one of the refractive index, the X-ray contrast property, or the water resistance.

According to Table 1, Examples 1-2 and 1-3 proved excellent physical properties (a low refractive index, a high X-ray contrast property, and a high water resistance) attributable to containing Rb even when containing Na and Cs.

According to Table 2, in Examples 2-1 to 2-4, the X-ray contrast property increased as the blending amount of Rb was increased, at the cost of only a slight increase in the refractive index. That is, it was revealed that increasing the blending amount of Rb could enhance the X-ray contrast property while inhibiting an increase in the refractive index. In other words, it was revealed that the X-ray contrast property could be desirably adjusted based on the blending amount of Rb.

According to Table 3, Examples 3-1 to 3-2 free of Na and K showed a good water resistance. On the other hand, Comparative Example 3-1 containing Na showed a bad water resistance. In other words, it was revealed that glass compositions containing Rb could have a further enhanced water resistance by being free of Na and/or K.

Furthermore, according to Tables 3 and 4, glass compositions containing Rb exhibited excellent physical properties (a low refractive index, a high X-ray contrast property, and a high water resistance) attributable to containing Rb even when combined with such elements as F, Zn, Sn, Sr, Zr, Ba, La, Gd, and the like. Especially, in Examples 3-1 to 3-2 and Examples 4-1 to 4-6, a further improvement in the X-ray contrast property attributable to containing Sn, Sr, Zr, Ba, La, and Gd was observed. In addition, in Examples 4-1 and 4-6, containing Sn, Zr, and La resulted in improvement in the X-ray contrast property and improvement in the water resistance as well. Moreover, containing F and Zn resulted in glass compositions having a low refractive index while exhibiting both a good X-ray contrast property and a sufficient water resistance.

The above-disclosed embodiments include, for example, the following aspects.

### (Clause 1)

A glass composition containing rubidium (Rb).

### (Clause 2)

The glass composition according to Clause 1, containing silicon (Si).

### (Clause 3)

The glass composition according to Clauses 1 or 2, containing cesium (Cs).

### (Clause 4)

The glass composition according to any one of Clauses 1 to 3, free of at least one selected from the group consisting of potassium (K) and sodium (Na).

### (Clause 5)

An X-ray contrast composition containing the glass composition of any one of Clauses 1 to 4.

### (Clause 6)

A dental composition containing the X-ray contrast composition of Clause 5.

Although the embodiments of the present invention have been described above, the present invention is not limited to the specific embodiments, and various modifications and changes are applicable within the scope of the invention described in the claims.

This application claims priority under Japanese Patent Application No. 2023-074448, filed on April 28, 2023, the entire contents of which are incorporated herein by reference.

## Claims

1. A glass composition, comprising:
rubidium (Rb).

2. The glass composition according to claim 1, comprising:
silicon (Si).

3. The glass composition according to claim 1, comprising:
cesium (Cs).

4. The glass composition according to claim 1,
wherein the glass composition is free of at least one selected from the group consisting of potassium (K) and sodium (Na).

5. An X-ray contrast composition, comprising:
the glass composition of any one of claims 1 to 4.

6. A dental composition, comprising:
the X-ray contrast composition of claim 5.
